Europäisches Patentamt

European Patent Office

Office européen des brevets

(19)

(11) Veröffentlichungsnummer: **0 327 040**
**A2**

(12) # EUROPÄISCHE PATENTANMELDUNG

(21) Anmeldenummer: 89101677.6

(22) Anmeldetag: 01.02.89

(51) Int. Cl.⁴: **A61K 31/135 , A61K 31/13 ,
A61K 31/48 , A61K 31/445 ,
A61K 31/405**

(30) Priorität: 05.02.88 YU 235/88

(43) Veröffentlichungstag der Anmeldung:
09.08.89 Patentblatt 89/32

(84) Benannte Vertragsstaaten:
**AT BE CH DE ES FR GB GR IT LI LU NL SE**

(71) Anmelder: **Sikiric, Predrag Dr.Sc.**
**Jurisiceva 5**
**41000 Zagreb(YU)**

Anmelder: **Rotkvic, Ivo Mr.Sc.**
**Cvjetno naselje 1 Nr. 21**
**Zagreb(YU)**

Anmelder: **Mise, Stjepan Mr.Sc.**
**Ruzveltova 37**
**Split(YU)**

Anmelder: **Udovicic, Ivan Dr.**
**S. Turudije 21**
**72270 Travnik(YU)**

Anmelder: **Krizanac, Simun, Dr.Sc.**
**Platana 8**
**41040 Zagreb(YU)**

Anmelder: **Jukic, Jerka, Dr.**
**Palih Omladinaca 33**
**Zagreb(YU)**

Anmelder: **Petek, Marijan Mr.Sc.**
**Visnjica 29**
**41000 Zagreb(YU)**

Anmelder: **Suchanek, Ernest, Dr.Sc.**
**Aleja V. Popovica 125**
**41000 Zagreb(YU)**

Anmelder: **Duvnjak, Marko Mr.Sc.**
**Rose Luksemburg 4**
**Zagreb(YU)**

(72) Erfinder: **Sikiric, Predrag Dr.Sc.**
**Jurisiceva 5**
**41000 Zagreb(YU)**
Erfinder: **Rotkvic, Ivo Mr.Sc.**
**Cvjetno naselje 1 Nr. 21**
**Zagreb(YU)**
Erfinder: **Mise, Stjepan Mr.Sc.**
**Ruzveltova 37**
**Split(YU)**
Erfinder: **Udovicic, Ivan Dr.**
**S. Turudije 21**
**72270 Travnik(YU)**
Erfinder: **Krizanac, Simun, Dr.Sc.**
**Platana 8**
**41040 Zagreb(YU)**
Erfinder: **Jukic, Jerka, Dr.**
**Palih Omladinaca 33**
**Zagreb(YU)**
Erfinder: **Petek, Marijan Mr.Sc.**
**Visnjica 29**
**41000 Zagreb(YU)**
Erfinder: **Suchanek, Ernest, Dr.Sc.**
**Aleja V. Popovica 125**
**41000 Zagreb(YU)**
Erfinder: **Duvnjak, Marko Mr.Sc.**
**Rose Luksemburg 4**
**Zagreb(YU)**

(74) Vertreter: **Gleiss, Alf-Olav, Dipl.-Ing. et al**
**Patentanwalt Silberburg Strasse 187**
**D-7000 Stuttgart 1(DE)**

(54) **Verwendung von Dopamin und/oder von Dopamin-Agonisten zur Herstellung eines Arzneimittels für die Behandlung des Verdauungstrakts.**

㊗ Es wird vorgeschlagen, Dopamin und/oder Dopamin-Agonisten zur Herstellung eines Arzneimittels für die Behandlung des Verdauungstrakts zu verwenden. Das Arzneimittel kann sowohl für die Behandlung der Schleimhaut des Verdauungstrakts, insbesondere bei Schädigungen durch nicht-steriode Schmerzmittel einge-setzt werden, als auch bei der Behandlung von Colitis ulcerosa, von Morbus Crohn sowie von Colitis pseudomembranacea.

## Verwendung von Dopamin und/oder von Dopamin-Agonisten zur Herstellung eines Arzneimittels für die Behandlung des Verdauungstrakts

Die Erfindung betrifft die Verwendung von Dopamin und/oder Dopamin-Agonisten zur Herstellung eines Arzneimittels für die Behandlung des Verdauungstrakts.

Derartige Arzneimittel werden insbesondere bei einer Schädigung der Schleimhaut des Verdauungstrakts, insbesondere bei Schädigungen durch nicht-steroide Schmerzmittel eingesetzt. Auch ist es möglich Dopamin und/oder Dopamin-Agonisten bei der Herstellung von Arzneimitteln zur Behandlung von Colitis ulcerosa, von Morbus Crohn, von Colitis pseudomembranacea oder von Schädigungen des Verdauungstrakts zu verwenden, die auf reduzierten Abwehrkräften des des Körpers beruhen.

Auch können Dopamin und/oder Dopamin-Agonisten zur Herstellung eines Arzneimittels verwendet werden durch welches die notwendige Dosierung von Corticosteroiden reduziert werden kann.

Überdies können mit den genannten Stoffen Arzneimittel hergestellt werden, die für die Behandlung von Nebenwirkungen von Corticosteroiden eingesetzt werden.

Für die Herstellung derartiger Arzneimittel werden insbesondere Bromocriptin und/oder Amantadin bevorzugt.

Bislang gibt es in den genannten Bereichen keine pharmazeutischen Therapiemöglichkeiten, wenn auch eine Möglichkeit der Behandlung von Komplikationen besteht, die aber außerhalb des pharmazeutischen Bereichs liegt.

Dopamin-Infusionen wurden Patienten verabreicht, die an Pankolitis litten, einer schweren Form der sich rasch ausbreitenden bzw. fulminanten Colitis ulcerosa. Derartige Infusionen wurden auch neben einer koventionellen Behandlung Patienten verabreicht, die an einer schweren Form von Morbus Crohn litten.

Bei der Untersuchung der technischen Probleme wurden die Wirkungen der Dopamin-Agonisten, Bromocriptin und Amantadin auf Schädigungen des Verdauungstraktes bei Ratten untersucht. Derartige Schädigungen wurden durch einzelne oder mehrfache Gaben von Indometacin hervorgerufen.

Bei den Dopamin-Infusionen für die an Pancolitis und an der schweren Form von Morbus Crohn leidenden Patienten wurde eine Dosierung von 3 $\mu$g/kg und pro Minute gewählt. Der Zustand der Patienten besserte sich innerhalb einer Woche. Es stellte sich auch heraus, daß die notwendige Dosis von Corticosteroiden im weiteren klinischen Verlauf reduziert werden konnte.

Bei einem Modellversuch wurden männliche und weibliche Albino-Wistar-Ratten verwendet, die ein Gewicht von 200 bis 300 g aufwiesen. Jede Gruppe umfaßte 8 bis 14 Ratten. Die untersuchten Substanzen wurden in einer Dosierung von 5,0 ml/kg verabreicht und zwar entweder einzeln oder in Kombination miteinander. Auch wurden zur Kontrolle identische Volumina einer physiologischen Kochsalzlösung verabreicht. Die untersuchten Substanzen wurden, ebenso wie die Lösung intraperitoneal verabreicht. Gleichzeitig wurde eine subcutane Injektion Indometacin verabreicht. Anschließend mußten die Tiere 24 Stunden fasten.

Tiere, denen eine Einzeldosis verabreicht wurde, wurden 24 Stunden nach Eingabe der Substanzen zur Untersuchung getötet. Bei mehrfacher Verabreichung des Mittels wurden die Ratten innerhalb von 24 Stunden zweimal behandelt, also an zwei aufeinanderfolgenden Tagen und dann 48 Stunden nach der zweiten Behandlung zur Untersuchung getötet. Gleichzeitig mit Indometacin, das mit einer Dosierung von 30,0 mg/kg subcutan verabreicht wurde, wurden folgende Substanzen angewandt:

a) Bromocriptin mit einer Dosierung von 2,5 und 5,0 mg/kg (intraperitoneal);

b) Amantadin in einer Dosierung von 20,0 mg/kg (intraperitoneal);

c) Haloperidol in einer Dosierung von 5,0 mg/kg (intraperitoneal);

d) Domperidon in einer Dosierung von 5,0 mg/kg (intraperitoneal);

e) Bromocriptin in einer Dosierung von 5,0 mg/kg dazu Haloperidol in einer Dosierung von 5,0 mg/kg;

f) Bromocriptin in einer Dosierung von 5,0 mg/kg dazu Domperidon in einer Dosierung von 5,0 mg/kg;

g) Amantadin in einer Dosierung von 20,0 mg/kg dazu Domperidon in einer Dosierung von 5,0 mg/kg;

h) Amantadin in einer Dosierung von 20,0 mg/kg mit Haloperidolin einer Dosierung von 5,0 mg/kg; schließlich

i) 0,9 % NaCl (intraperitoneal) zur Kontrolle.

Die Schädigungen des Verdauungstrakts wurden dadurch hervorgerufen, daß

a) Indometacin einmal subcutan verabreicht wurde oder

b) Indometacin zweimal subcutan in einem Abstand von 24 Stunden verabreicht wurde.

Bei der klinischen Überwachung der Tiere stellte sich heraus, daß die mit den Dopamin-Agonisten Bromocriptin oder Amantadin behandelten Tiere sich in einem besseren Allgemeinzustand befanden, als die, die gemeinsam mit Indometacin zur Kontrolle die physiologische Kochsalzlösung oder Dopamin-Antagonisten Haloperidol oder Domperidon gemeinsam mit Dopamin, oder die Agonisten Bromocriptin oder Amantadin oder Dopamin-Antagonisten allein erhielten. Meläna wurde bei mit Indometacin behandelten Tiere am meisten beobachtet, sofern Dopamin-Antagonisten verabreicht wurden; weniger trat dieses Phänomen bei der Kontrollgruppe und in der Gruppe auf, die mit Dopamin-Antagonisten und mit Dopamin-Agonisten behandelt wurden. Am wenigsten trat Meläna in der Gruppe auf, die ausschließlich mit Dopamin-Agonisten behandelt wurde.

Bei der patho-histologischen Ausarbeitung bzw. Auswertung wurde nach dem Tod der Tiere eine makroskopische und mikroskopische Untersuchung des gesamten Verdauungstrakts durchgeführt. Die Ergebnisse sind in den Tabellen 1 bis 3 dargestellt. Nach einer einzigen Gabe von Indometacin wurden beträchtliche Schädigungen bei den Ratten der Kontrollgruppe festgestellt und zwar sowohl im Magen als auch im Darm. Nach mehrfacher Verabreichung waren die Schäden im Magen wesentlich geringer und die im Darm wesentlich deutlicher ausgeprägt.

Durch Dopamin-Agonisten, Bromocriptin und Amantadin, wurden die Schädigungen aufgrund von Indometacin wesentlich reduziert, während durch Dopamin-Antagonisten, Haloperidol und Domperidon Schädigungen verstärkt wurden. Bei gleichzeitiger Anwendung wurde durch die Dopamin-Agonisten die nozizeptive Wirkung der Dopamin-Antagonisten gemildert oder auch aufgehoben, ebenso deren antagonistische Wirkung auf den zytoprotektiven Effekt bei Magenschädigungen, die bei wiederholter Anwendungen von Indometacinen auftraten.

Im folgenden wird der Wirkungsmechanismus genauer beschrieben:

Die Schlüsselrolle der Schleimhaut bei der Reduktion der Durchblutung und bei der Vasoconstriktion infolge einer Schädigung der Schleimhaut des Verdauungstrakts ist gut bekannt. Es ist auch bekannt, daß Dopamin bei der Schleimhaut sowohl des Magens als auch des Darms vasodilatorisch wirkt und die Blutströmung bzw. den Blutfluß um ein Mehrfaches verstärkt. Man kann davon ausgehen, daß eine derartige Wirkung von Dopamin eine wahrnehmbare und schnelle Verbesserung des klinischen Bildes bei Patienten bewirkt, die unter einer ernsten, fulminanten Pancolitis, beispielsweise unter Morbus Crohn leiden. Es ist bekannt, daß Amantadin die Freisetzung von Dopamin in den präsynaptischen Vesikeln bzw. Bläschen steigert, und daß Bromocriptin als Agonist für Dopamin-Rezeptoren wirkt. Bekannt ist auch, daß Haloperidol spezifisch die zentralen Dopamin-Rezeptoren, und daß Domperidon die peripheren Dopamin Rezeptoren blockiert. Daraus kann geschlossen werden, daß die beobachteten Wirkungen wahrscheinlich auf dem Einfluß der zentalen und peripheren Dopamin-Mechanismen auf die Zirkulation des Verdauungstrakts beruhen.

Zu den Tabellen 1 bis 3 ist folgendes festzuhalten:

Tabelle 1 betrifft die Geschwürbildung bei einmaliger und mehrfacher Gabe von Indometacin (30 mg/kg; subcutan), das gleichzeitig mit physiologischer Kochsalzlösung, Bromocriptin, Amantadin und Domperidon oder Haloperidol bei Schleimhautschädigungen im Magen- oder Darmbereich verabreicht wird. Bei den Versuchen, bei denen den Versuchstieren eine einmalige Gabe verabreicht wurde, wurden diese 24 Stunden nach Eingabe der Mittel getötet. Die anderen Tiere wurden an zwei aufeinanderfolgenden Tagen beispielsweise um 10 Uhr behandelt und 48 Stunden nach der zweiten Behandlung getötet. In Tabelle 1/1 sind die Untersuchungsergebnisse, die den Magen betreffen, wieder gegeben, in Tabelle 1/2 die Ergebnisse der Darmuntersuchungen.

In Tabelle 2 ist die histologische Auswertung (Nr. 0 bis 6) der Wirkung auf Geschwüre bei ein- oder mehrmaliger Verabreichung von Indometacin (30 mg/kg; subcutan) untersucht, das gleichzeitig mit physiologischer Kochsalzlösung, Bromocriptin, Amantadin und Domperidon oder Haloperidol bei Schädigungen der Magenschleimhaut verabreicht wurde. Das Versuchsprotokoll entspricht im übrigen dem von Tabelle 1. Tabelle 2/1 gibt das Untersuchungsergebnis bei einmaliger, Tabelle 2/2 bei mehrfacher Behandlung der Tiere wieder.

Tabelle 3 zeigt die histologische Auswertung (Nr. 0 bis 4) der Wirkung auf Geschwüre bei ein- und mehrfacher Gabe von Indometacin (30 mg/kg; subcutan), das gleichzeitig mit physiologischer Kochsalzlösung, Bromocriptin, Amantadin und Domperidon oder Haloperidol bei Schleimhautschädigungen des Darms verabreicht wurde. Das Versuchsprotokoll entspricht im übrigen dem von Tabelle 1. Tabelle 3/1 gibt das Untersuchungsergebnis bei einmaliger, Tabelle 3/2 bei mehrfacher Behandlung der Tiere wieder.

Tabelle 1/1

Summe der Durchmesser der größten Geschwüre in mm

| Behandlung | mg/kg i.p. | Magen | Anzahl der Ratten |
|---|---|---|---|
| **Einmalige Behandlung** | | | |
| Physiologische Kochsalzlösung (Kontrolle) | | 22.9± 5.1 | 11 |
| Bromocriptin | 2.5 | 7.8± 3.3[a] | 12 |
| | 5.0 | 6.5± 1.6[b] | 11 |
| Amantadin | 20.0 | 7.1± 2.5[a] | 10 |
| Domperidon | 5.0 | 55.3± 5.1[c] | 14 |
| Domperidon | 5.0 | | |
| + Bromocriptin | 5.0 | 6.7± 2.5[a,f] | 11 |
| + Amantadin | 20.0 | 6.0± 2.2[b,f] | 8 |
| Haloperidol | 5.0 | 43.1± 1.7[b] | 8 |
| Haloperidol | 5.0 | | |
| + Bromocriptin | 5.0 | 6.5± 2.9[a,h] | 8 |
| + Amantadin | 20.0 | 6.0± 2.4[b,h] | 8 |
| **Mehrfachbehandlung** | | | |
| Physiologische Kochsalzlösung (Kontrolle) | 5.0 | 2.9± 0.9[c] | 8 |
| Bromocriptin | 5.0 | 0 ± 0[i] | 8 |
| Amantadin | 20.0 | 0 ± 0[i] | 8 |
| Domperidon | 5.0 | 87.5± 1.8[f,j] | 8 |
| Domperidon | 5.0 | | |
| + Bromocriptin | 5.0 | 0 ± 0[i,k] | 8 |
| + Amantadin | 20.0 | 0 ± 0[i,k] | 8 |
| Haloperidol | 5.0 | 73.6± 8.8[j,g] | 8 |
| Haloperidol | 5.0 | | |
| + Bromocriptin | 5.0 | 0 ± 0[i,m] | 8 |
| + Amantadin | 20.0 | 0 ± 0[i,m] | 8 |

$\bar{x}$ ± S.E.M. (Standard-Fehlerrate)

**Einmalige Behandlung:**
t-test versus:
Kontrollgruppe [a] $p < 0.05$, [b] $p < 0.001$;
Domperidon: [f] $p < 0.001$,
Haloperidol: [g] $p < 0.01$, [h] $p < 0.001$

**Mehrfachbehandlung:**
t-test versus:
Kontrollgruppe [i] $p < 0.01$; [j] $p < 0.001$
Domperidon: [k] $p < 0.001$,
Haloperidol: [m] $p < 0.001$

## Tabelle 1/2

Summe der Durchmesser der größten Geschwüre in mm

| Behandlung | mg/kg i.p. | Darm | Anzahl der Ratten |
|---|---|---|---|
| **Einmalige Behandlung** | | | |
| Physiologische Kochsalzlösung (Kontrolle) | | $8.6 \pm 2.6$ | 12 |
| Bromocriptin | 2.5 | $1.7 \pm 0.7^a$ | 12 |
| | 5.0 | $0.7 \pm 0.3^b$ | 11 |
| Amantadin | 20.0 | $1.0 \pm 0.4^b$ | 11 |
| Domperidon | 5.0 | $161.3 \pm 50.6^b$ | 14 |
| Domperidon | 5.0 | | |
| + Bromocriptin | 5.0 | $0.6 \pm 0.2^{b,f}$ | 11 |
| + Amantadin | 20.0 | $2.1 \pm 1.0^{a,f}$ | 8 |
| Haloperidol | 5.0 | $422.1 \pm 9.5^c$ | 8 |
| Haloperidol | 5.0 | | |
| + Bromocriptin | 5.0 | $1.0 \pm 0.3^{b,h}$ | 8 |
| + Amantadin | 20.0 | $1.3 \pm 0.5^{a,h}$ | 8 |
| **Mehrfachbehandlung** | | | |
| Physiologische Kochsalzlösung (Kontrolle) | 5.0 | $207.5 \pm 58.6^b$ | 8 |
| Bromocriptin | 5.0 | $0 \pm 0^j$ | 8 |
| Amantadin | 20.0 | $0 \pm 0^j$ | 8 |
| Domperidon | 5.0 | $500.0 \pm 40.1^{f,j}$ | 8 |
| Domperidon | 5.0 | | |
| + Bromocriptin | 5.0 | $0 \pm 0^{j,k}$ | 8 |
| + Amantadin | 20.0 | $0 \pm 0^{j,k}$ | 8 |
| Haloperidol | 5.0 | $724.3 \pm 14.6^{j,h}$ | 8 |
| Haloperidol | 5.0 | | |
| + Bromocriptin | 5.0 | $107.8 \pm 11.3^m$ | 8 |
| + Amantadin | 20.0 | $125.0 \pm 23.2^m$ | 8 |

$\bar{x} \pm$ S.E.M. (Standard-Fehlerrate)

Einmalige Behandlung:
t-test versus:
Kontrollgruppe $^a p < 0.05$, $^b p < 0.001$;
Domperidon: $^f p < 0.001$,
Haloperidol: $^g p < 0.01$, $^h p < 0.001$

Mehrfachbehandlung:
t-test versus:
Kontrollgruppe $^i p < 0.01$; $^j p < 0.001$
Domperidon: $^k p < 0.001$,
Haloperidol: $^m p < 0.001$

6

## Tabelle 2/1

| Behandlung | mg/kg i.p. | 0 | 1 | 2 | 3 | 4 | 5 | 6 | n |
|---|---|---|---|---|---|---|---|---|---|
| **Einmalige Behandlung** Nr. | | | | | | | | | |
| Physiologische Kochsalzlösung (Kontrolle) | | – | – | – | 3 | 3 | 3 | 2 | 11 |
| Bromocriptin | 2.5 | 2 | 8 | 2 | – | – | – | – | 12[b] |
| Bromocriptin | 5.0 | 2 | 7 | 2 | – | – | – | – | 11[b] |
| Amantadin | 20.0 | 3 | 5 | 2 | – | – | – | – | 10[b] |
| Domperidon | 5.0 | – | – | – | – | – | 8 | 6 | 14[a] |
| Domperidon | 5.0 | | | | | | | | |
| + Bromocriptin | 5.0 | 2 | 7 | 2 | – | – | – | – | 11[b,d] |
| + Amantadin | 20.0 | 2 | 4 | 2 | – | – | – | – | 8[b,d] |
| Haloperidol | 5.0 | – | – | – | – | – | 4 | 4 | 8 |
| Haloperidol | 5.0 | | | | | | | | |
| + Bromocriptin | 5.0 | 2 | 5 | – | – | – | – | – | 8[b,e] |
| + Amantadin | 20.0 | 2 | 3 | 3 | – | – | – | – | 8[b,e] |

Kruskal-Wallis-Test : [c]$p < 0.05$

Mann-Whitney U-Test versus
zugehörige Kontrollgruppe : [a]$p < 0.05$, [b]$p < 0.01$
Domperidon : [d]$p < 0.01$
Haloperidol : [e]$p < 0.01$

## Tabelle 2/2

| Behandlung | mg/kg i.p. | Mehrfache Behandlung Nr. | | | | | | | n |
|---|---|---|---|---|---|---|---|---|---|
| | | 0 | 1 | 2 | 3 | 4 | 5 | 6 | |
| Physiologische Kochsalzlösung (Kontrolle) | | 4 | 3 | 1 | − | − | − | − | 8 |
| Bromocriptin | 2.5 | | | | | | | | |
| Bromocriptin | 5.0 | 8 | − | − | − | − | − | − | 8 |
| Amantadin | 20.0 | 8 | − | − | − | − | − | − | 8 |
| Domperidon | 5.0 | − | − | − | − | − | − | 8 | $8^b$ |
| Domperidon | 5.0 | | | | | | | | |
| + Bromocriptin | 5.0 | 8 | − | − | − | − | − | − | $8^d$ |
| + Amantadin | 20.0 | 8 | − | − | − | − | − | − | $8^d$ |
| Haloperidol | 5.0 | − | − | − | − | − | 1 | 7 | $8^b$ |
| Haloperidol | 5.0 | | | | | | | | |
| + Bromocriptin | 5.0 | 8 | − | − | − | − | − | − | $8^e$ |
| + Amantadin | 20.0 | 8 | − | − | − | − | − | − | $8^e$ |

Kruskal-Wallis-Test : $^c p < 0.05$

Mann-Whitney U-Test versus
zugehörige Kontrollgruppe : $^a p < 0.05$, $^b p < 0.01$
Domperidon : $^d p < 0.01$
Haloperidol : $^e p < 0.01$

## Tabelle 3/1

| Behandlung | mg/kg i.p. | Einmalige Behandlung Nr. | | | | | |
|---|---|---|---|---|---|---|---|
| | | 0 | 1 | 2 | 3 | 4 | n |
| Physiologische Kochsalzlösung (Kontrolle) | | 3 | 5 | 3 | – | 1 | 12 |
| Bromocriptin | 2.5 | 8 | 4 | – | – | – | 12[a] |
| pBromocriptin | 5.0 | 8 | 3 | – | – | – | 11[b] |
| Amantadin | 20.0 | 8 | 3 | – | – | – | 11[b] |
| Domperidon | 5.0 | – | – | – | 10 | 4 | 14[b] |
| Domperidon | 5.0 | | | | | | |
| + Bromocriptin | 5.0 | 8 | 3 | – | – | – | 11[b,c] |
| + Amantadin | 20.0 | 6 | 3 | – | – | – | 8[a,d] |
| Haloperidol | 5.0 | – | – | – | 4 | 4 | 8 |
| Haloperidol | 5.0 | | | | | | |
| + Bromocriptin | 5.0 | 6 | 2 | – | – | – | 8[a,f] |
| + Amantadin | 20.0 | 6 | 2 | – | – | – | 8[a,f] |

Kruskal-Wallis-Test : [c]$p < 0.05$

Mann-Whitney U-Test versus
zugehörige Kontrollgruppe : [a]$p < 0.05$, [b]$p < 0.01$
Domperidon : [d]$p < 0.01$
Haloperidol : [e]$p < 0.01$, [f]$p < 0.01$
Amantadin : [g]$p < 0.01$
Bromocriptin : [h]$p < 0.01$

## Tabelle 3/2

| Behandlung | mg/kg i.p. | Mehrfache Behandlung Nr. | | | | | |
|---|---|---|---|---|---|---|---|
| | | 0 | 1 | 2 | 3 | 4 | n |
| Physiologische Kochsalzlösung (Kontrolle) | | – | – | 1 | 3 | 4 | 8 |
| Bromocriptin | 2.5 | | | | | | |
| Bromocriptin | 5.0 | 8 | – | – | – | – | $8^b$ |
| Amantadin | 20.0 | 8 | – | – | – | – | $8^b$ |
| Domperidon | 5.0 | – | – | – | – | 8 | 8 |
| Domperidon | 5.0 | | | | | | |
| + Bromocriptin | 5.0 | 8 | – | – | – | – | $8^d$ |
| + Amantadin | 20.0 | 8 | – | – | – | – | $8^{b,d}$ |
| Haloperidol | 5.0 | – | – | – | – | 8 | 8 |
| Haloperidol | 5.0 | | | | | | |
| + Bromocriptin | 5.0 | – | – | 2 | 3 | 3 | $8^{e,h}$ |
| + Amantadin | 20.0 | – | – | 2 | 3 | 3 | $8^{e,g}$ |

Kruskal-Wallis-Test : $^c p < 0.05$

Mann-Whitney U-Test versus
zugehörige Kontrollgruppe : $^a p < 0.05$, $^b p < 0.01$
Domperidon : $^d p < 0.01$
Haloperidol : $^e p < 0.01$, $^f p < 0.01$
Amantadin : $^g p < 0.01$
Bromocriptin : $^h p < 0.01$

**Ansprüche**

1. Verwendung von Dopamin und/oder Dopamin-Agonisten zur Herstellung eines Arzneimittels für die Behandlung des Verdauungstrakts.

2. Verwendung von Dopamin und/oder Dopamin-Agonisten zur Herstellung eines Arzneimittels nach Anspruch 1 für die Behandlung der Schleimhaut des Verdauungstrakts, insbesondere bei Schädigungen durch nichtsteroide Schmerzmittel.

3. Verwendung von Dopamin und/oder Dopamin-Agonisten zur Herstellung eines Arzneimittels nach Anspruch 1 für die Behandlung von Colitis ulcerosa.

4. Verwendung von Dopamin und/oder Dopamin-Agonisten zur Herstellung eines Arzneimittels nach Anspruch 1 für die Behandlung von Morbus Crohn.

5. Verwendung von Dopamin und/oder Dopamin-Agonisten zur Herstellung eines Arzneimittels nach Anspruch 1 für die Behandlung von Colitis pseudomembranacea.

6. Verwendung von Dopamin und/oder Dopamin-Agonisten zur Herstellung eines Arzneimittels nach Anspruch 1 für die Behandlung des Verdauungstrakts bei Schädigungen, die auf reduzierten Abwehrkräften des Körpers beruhen.

7. Verwendung von Dopamin und/oder Dopamin-Agonisten zur Herstellung eines Arzneimittels nach Anspruch 1, durch welches die notwendige Dosierung von Corticosteroiden reduziert wird.

8. Verwendung von Dopamin und/oder Dopamin-Agonisten zur Herstellung eines Arzneimittels nach Anspruch 1 für die Behandlung von Nebenwirkungen von Corticosteroiden.

9. Verwendung von Bromocriptin und/oder Amantadin zur Herstellung eines Arzneimittels für die Behandlung des Verdauungstrakts nach einem der Ansprüche 1 bis